# FASCICULE DE BREVET EUROPEEN

(11) **EP 0 738 508 B1**
(45) Date de publication et mention de la délivrance du brevet: **25.06.1997**
(21) Numéro de dépôt: 96400512.8
(22) Date de dépôt: 12.03.1996
(51) Int. Cl.: A61K 7/42, A61K 7/48

(54) **Composition pour lutter contre les taches et/ou le vieillissement de la peau, ses utilisations**
Hautdepigmentierungs- und/oder Alterungsschutzmittel und seine Verwendungen
Skin whitening and/or anti ageing composition and its uses

(30) Priorité: 20.04.1995 FR 9504748
(43) Date de publication de la demande: 23.10.1996
(73) Titulaire: L'OREAL, 75008 Paris (FR)
(72) Inventeur: Simon, Pascal, F-94400 Vitry-Sur-Seine (FR); Candau, Didier, F-91570 Bievres (FR)
(74) Mandataire: Lhoste, Catherine

(56) Documents cités:
- EP-A- 0 398 484
- EP-A- 0 425 066
- EP-A- 0 457 687
- EP-A- 0 487 404
- EP-A- 0 518 772
- EP-A- 0 531 192
- GB-A- 2 200 552
- GB-A- 2 225 013
- PATENT ABSTRACTS OF JAPAN vol. 11, no. 214 (C-434) [2661] , 10 Juillet 1987 & JP-A-62 033195 (RIYUUHOUDOU SEIYAKU K.K.), 13 Février 1987,
- PATENT ABSTRACTS OF JAPAN vol. 8, no. 114 (C-225) [1551] , 26 Mai 1984 & JP-A-59 027825 (SUNSTAR HAMIGAKI K.K.), 14 Février 1984,
- DATABASE WPI Week 9338 24 Août 1993 Derwent Publications Ltd., London, GB; AN 93-299537 XP002000829 & JP-A-05 213 736 (UNITIKA LTD)

## Description

L'invention se rapporte à une composition cosmétique et/ou dermatologique destinée à prévenir et/ou lutter contre les taches et/ou le vieillissement cutanés. Cette composition se présente sous forme d'une crème blanche onctueuse pouvant être appliquée sur le visage, le corps, le cou, les mains et/ou les jambes d'êtres humains.

L'invention se rapporte aussi à une utilisation de cette composition pour le traitement cosmétique de la peau, à l'utilisation de cette composition pour la préparation d'une crème destinée au traitement dermatologique de la peau et à un procédé de traitement cosmétique.

Au cours du processus de vieillissement, il apparaît différents signes sur la peau, très caractéristiques de ce vieillissement, se traduisant notamment par une modification de la structure et des fonctions cutanées. Ce vieillissement est de nature physiologique mais il peut être également photoinduit, c'est-à-dire dû à une exposition répétée de la peau à la lumière solaire, notamment ultraviolette. L'action de cette lumière sur les constituants de la peau et sur le sébum sécrété par la peau entraîne en particulier la formation de radicaux libres oxygénés. Or, ces radicaux provoquent des dégats importants, notamment dans les membranes cellulaires (perméabilité des membranes), les noyaux des cellules (mutation par action sur l'ARN ou ADN), et les tissus (nécroses, dégénérescence) ; il est donc nécessaire de protéger la peau contre ces radicaux libres.

Les principaux signes cliniques du vieillissement cutané sont notamment l'apparition de ridules et de rides profondes, en augmentation avec l'âge, ainsi qu'une désorganisation du "grain" de la peau ; autrement dit, le microrelief cutané est moins régulier et présente un caractère anisotrope.

Par ailleurs, le teint de la peau est généralement modifié et apparaît plus jaune ; ceci semble être dû essentiellement à une désorganisation de la microcirculation (moins d'hémoglobine au niveau du derme papillaire). De plus, de nombreuses taches colorées et/ou plus foncées apparaissent à la surface de la peau, et notamment sur les mains, conférant à la peau une hétérogénéité. En général, ces taches sont dues à une production importante de mélanine dans l'épiderme et/ou le derme de la peau. Dans certains cas d'exposition intense aux rayons solaires, ces taches peuvent devenir cancéreuses. Par ailleurs, il peut exister sur certaines zones de la peau des irritations diffuses et parfois des télangiectasies.

Un autre signe clinique du vieillissement est l'aspect sec et rêche de la peau qui est dû essentiellement à une desquamation plus importante ; ces squames, en diffractant les rayons lumineux, participent aussi à l'aspect un peu gris du teint.

On constate enfin une perte de fermeté et de tonicité de la peau qui, comme pour les rides et les ridules, s'explique du moins en partie par une atrophie dermique et épidermique ainsi qu'un applatissement de la formation dermoépidermique ; la peau est moins épaisse et plus flasque, et l'épaisseur de l'épiderme diminue.

On constate donc que les signes cliniques du vieillissement cutané résultent essentiellement d'un dysfonctionnement des principaux mécanismes biologiques intervenant au niveau de la peau.

Aussi, la composition selon l'invention est une composition apte à prévenir et/ou lutter contre l'apparition du vieillissement et les signes de vieillissement existants, comme les rides, les ridules, à prévenir et/ou lutter contre les taches de pigmentation de la peau, quelle que soit leur origine, ainsi qu'à protéger la peau notamment par suppression de la formation de radicaux libres oxygénés.

Un des moyens connus pour lutter contre le vieillissement prématuré de la peau consiste à apporter sur la peau des molécules capables d'aider les cellules à se défendre contre l'excès de radicaux libres photo-induits. Un des moyens efficaces de lutte contre ces radicaux libres est l'utilisation d'un filtre solaire absorbant le rayonnement UVA (320 nm à 400 nm). Ce filtre peut être lipophile ou hydrophile.

En vue de fabriquer une grande variété de compositions stables à usage topique, l'invention s'intéresse aux filtres hydrophiles ou hydrosolubles, les filtres lipophiles ou liposolubles limitant l'emploi d'excipients.

Comme filtres hydrosolubles absorbant les UVA, on connaît l'Uvinul® MS-40 de BASF qui est l'acide hydroxy-2-méthoxy-4-benzophénone-5 sulfonique, les dérivés sulfoniques du benzylidènecamphre comme l'acide benzène 1,4 [di(3-méthylidène-campho 10-sulfonique)], appelé aussi (selon la nomenclature CTFA - 5ème édition) l'acide téréphtalylidène - di-camphosulfonique.

Pour une lutte plus efficace contre les radicaux libres, il est intéressant d'associer aux filtres UVA des molécules capables de bloquer les réactions en chaîne des radicaux libres avant les étapes ultimes de dégradations des constituants biologiques de la peau (lipides, protéines et acides nucléiques). Ces molécules sont en particulier des agents anti-oxydants et/ou antiradicaux libres.

L'une des molécules connues pour présenter un puissant pouvoir réducteur hydrophile et pour réagir avec les radicaux libres commes les radicaux peroxyde, superoxyde et hydroxyle est la vitamine C. Malheureusement, cette vitamine est très instable en milieu aqueux et ne peut donc pas être associée aux filtres sulfoniques hydrosolubles cités précédemment.

L'invention a justement pour objet une composition pour application topique présentant un fort pouvoir protecteur contre les radicaux photo-induits permettant de prévenir et/ou lutter efficacement contre le vieillissement de la peau et/ou les taches cutanées extrinsèques et intrinséques (physiologiques), pouvant se présenter sous diverses formes galéniques.

Selon une caractéristique essentielle de l'invention, cette composition contient, dans un milieu cosmétiquement et/ou dermatologiquement, acceptable au moins un ester d'ose de l'acide ascorbique et au moins un filtre UVA sulfonique hydrosoluble.

Ainsi, de façon surprenante, la demanderesse a découvert que les dérivés d'ose de l'acide ascorbique étaient parfaitement compatibles avec les filtres UVA sulfoniques hydrosolubles et qu'ils pouvaient être formulés dans tout type d'excipient contenant de l'eau. Ces dérivés présentent notamment l'avantage d'être très solubles à l'eau et de ne pas modifier les propriété physiques et chimiques de ces filtres. De plus ces dérivés sont bioconvertibles en vitamine C par les enzymes correspondantes de la peau.

La composition de l'invention peut avantageusement se présenter sous toutes les formes galéniques normalement utilisées pour une application topique telles que les solutions, les gels aqueux ou hydroalcooliques, les émulsions huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques. Ainsi, la composition de l'invention peut se présenter sous forme d'une crème, d'une pommade, d'une lotion ou d'un sérum.

La composition permet d'atténuer efficacement les rides et les ridules, de modifier le teint de la peau qui apparaît plus rosé, d'effacer les taches de pigmentation, de supprimer les squames et de donner une consistance plus élastique à la peau. Elle permet un protection efficace de la peau contre les rayons solaires ainsi qu'un blanchiment de la peau.

Les esters d'ose de l'acide ascorbique utilisables dans l'invention sont notamment les dérivés glycosylé, mannosylé, fructosylé, fucosylé, galactosylé N-acétylglucosaminé, N-acétylmuramique de l'acide ascorbique et leurs mélanges et plus spécialement l'ascorbyl-2 glucoside ou 2-O-α-D glucopyranosyl de l'acide L ascorbique ou encore le 6-O-β-D galactopyranosyl de l'acide L ascorbique. Ces derniers composés ainsi que leurs fabrications sont en particulier décrits dans les documents EP-A-487404, EP-A-425066 et J05213736.

Les filtres UVA utilisables dans l'invention sont tous les filtres sulfonés et/ou sulfonatés hydrosolubles. Ces filtres peuvent être partiellement neutralisés par une base organique comme la triétanolamine, l'éthylène diamine.

De façon avantageuse les filtres de l'invention sont les dérivés sulfonés ou sulfonatés du benzylidène camphre mais on peut aussi utiliser les dérivés sulfonés ou sulfonatés de la benzophénone.

En particulier, les dérivés de benzylidène camphre utilisables dans l'invention présente la formule générale (a) suivante : 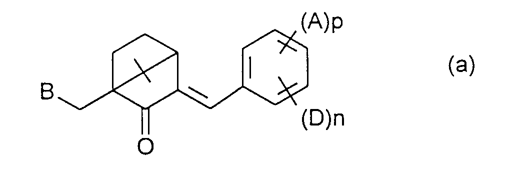 dans laquelle :
B représente -H ou -SO₃H,
0 ≤ p ≤ 1 avec B = -SO₃H quand p = 0,
0 ≤ n ≤ 4,
D représente un ou plusieurs radicaux alkyle ou alcoxy, identiques ou différents quand n ≥ 2, linéaires ou ramifiés contenant de 1 à 18 atomes de carbone environ, un radical halogéno, un radical hydroxyle.

A, de préférence en méta ou en para, représente
soit un radical SO₃H ;
soit un groupement : 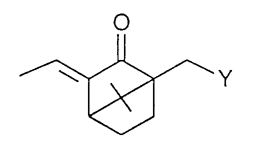 dans lequel Y représente H ou SO₃H ;
soit un groupement : 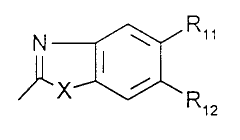 dans lequel :
R₁₁ désigne un atome d'hydrogène, un radical alkyle ou alcoxy, linéaire ou ramifié contenant de 1 à 6 atomes de carbone environ ou le radical -SO₃H, R₁₁ étant -SO₃H lorsque B = -H,
R₁₂ désigne un atome d'hydrogène, un radical alkyle ou alcoxy, linéaire ou ramifié, contenant de 1 à 6 atomes de carbone environ,
X est un atome d'oxygène, de soufre ou un groupement -NR-, R étant un atome d'hydrogène ou un radical alkyle, linéaire ou ramifié, contenant de 1 à 6 atomes de carbone environ,
   et dans laquelle au moins une fonction -SO₃H est éventuellement neutralisée.

On peut citer comme exemples particuliers de composés de formule (a) les dérivés de formules (I), (II), (III) suivantes :

### Formule (I) :

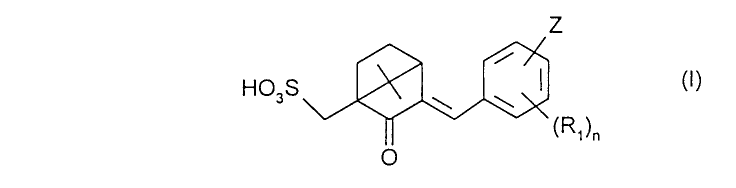

dans laquelle :
- Z, de préférence en position para ou méta, désigne un groupement 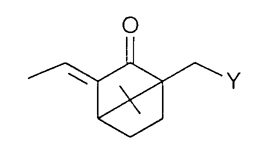 dans lequel Y représente -H ou -SO₃H, éventuellement neutralisé,
- n est égal à 0 ou est un nombre allant de 1 à 4 (0 ≤n≤ 4),
- R₁, représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0 , Z en position para et Y = -SO₃H : l'acide benzène 1,4 [di(3-méthylidène-campho 10-sulfonique)], appelé aussi (selon la nomenclature CTFA - 5ème édition) l'acide téréphtalylidène - di-camphosulfonique.

### Formule (II) :

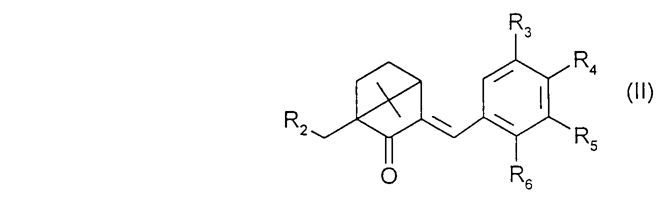

dans laquelle :
- R₂ désigne un atome d'hydrogène ou un radical -SO₃H,
- R₃, R₄, R₅ et R₆, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno ; de plus un radical R₃ à R₆ seulement peut être un radical
- SO₃H, au moins un des radicaux R₃ à R₆ désignant le radical -SO₃H quand R₂ est un atome d'hydrogène. Une ou plusieurs fonctions -SO₃H peuvent aussi être neutralisées.

On peut citer comme exemples particuliers les composés suivants de formule (II) dans laquelle :
- R₄ désigne le radical -SO₃H en position para du benzylidènecamphre et R₂, R₃, R₅ et R₆ désignent chacun un atome d'hydrogène, c'est-à-dire l'acide 4'-sulfo 3-benzylidènecamphre.
- R₃, R₄, R₅ et R₆ désignent chacun un atome d'hydrogène et R₂ désigne un radical -SO₃H, c'est-à-dire l'acide 3-benzylidène campho-10 sulfonique.
- R₄ désigne un radical méthyle en position para du benzylidènecamphre, R₅ un radical -SO₃H et R₂, R₃ et R₆ représentent un atome d'hydrogène, c'est-à-dire l'acide 4'-méthyl 3'-sulfo 3-benzylidènecamphre.
- R₄ désigne un atome de chlore en position para du benzylidènecamphre, R₅ un radical -SO₃H et R₂, R₃ et R₆ représentent un atome d'hydrogène, c'est-à-dire l'acide 4'-chloro 3'-sulfo 3-benzylidènecamphre.
- R₄ désigne un radical méthyle en position para du benzylidènecamphre, R₃, R₅ et R₆ désignent un atome d'hydrogène et R₂ désigne un radical -SO₃H, c'est-à-dire l'acide 4'-méthyl 3-benzylidène campho 10-sulfonique.
- R₂ représente un radical -SO₃H, R₃ est un radical méthyle, R₄ un atome d'hydrogène, R₅ un radical tertiobutyle, R₆ un radical hydroxyle, c'est-à-dire l'acide (3-t-butyl 2-hydroxy 5-méthyl) 3-benzylidène campho-10-sulfonique.
- R₂ représente un radical -SO₃H, R₃ est un radical méthoxy, R₄ un atome d'hydrogène, R₅ un radical tertiobutyle, R₆ un radical hydroxyle, c'est-à-dire l'acide (3-t-butyl 2-hydroxy 5-méthoxy) 3-benzylidène campho-10-sulfonique.
- R₂ représente un radical -SO₃H, R₃ et R₅ désignent chacun un radical tertiobutyle, R₄ un radical hydroxyle, R₆ un atome d'hydrogène, c'est-à-dire l'acide (3,5-diterbutyl 4-hydroxy) 3-benzylidène campho-10-sulfonique.
- R₄ représente un radical méthoxy en para, R₅ représente -SO₃H, les radicaux R₂, R₃ et R₆ représentent H, c'est-à-dire l'acide 4'-méthoxy 3'-sulfo-3-benzylidène camphre.
- R₂ désigne un radical -SO₃H, R₃ et R₆ représentent H, R₄ et R₅ formant un radical méthylènedioxy, c'est-à-dire l'acide 3-(4,5-méthylènedioxy) benzylidène campho-10-sulfonique.
- R₂ représente un radical -SO₃H, R₄ un radical méthoxy et les radicaux R₃, R₅ et R₆ représentent H, c'est-à-dire l'acide 3-(4-méthoxy) benzylidène campho-10-sulfonique.
- R₂ représente un radical -SO₃H, R₄ et R₅ sont tous deux un radical méthoxy et les radicaux R₃ et R₆ représentent H, c'est-à-dire l'acide 3-(4,5-diméthoxy) benzylidène campho-10-sulfonique.
- R₂ représente un radical -SO₃H, R₄ est un radical n-butoxy et les radicaux R₃, R₅ et R₆ représentent un atome d'hydrogène, c'est-à-dire l'acide 3-(4-n.butoxy) benzylidène campho-10-sulfonique.
- R₂ représente un radical -SO₃H, R₄ est un radical n-butoxy, R₅ est un radical méthoxy et R₃ et R₆ désignent tous deux un atome d'hydrogène, c'est-à-dire l'acide 3-(4-n.butoxy 5-méthoxy) benzylidène campho-10-sulfonique.

### Formule (III) :

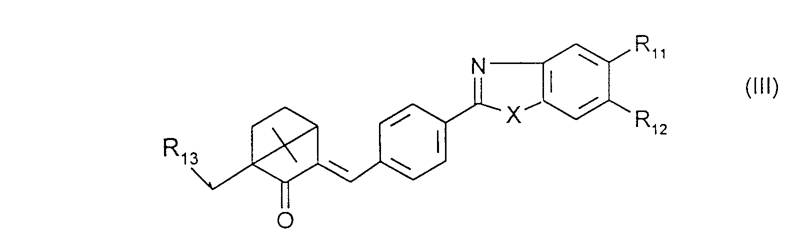

dans laquelle :
- R₁₁ désigne un atome d'hydrogène, un radical alkyle ou alcoxy, linéaire ou ramifié, contenant de 1 à 6 atomes de carbone environ ou un radical -SO₃H,
- R₁₂ désigne un atome d'hydrogène, un radical alkyle ou alcoxy, linéaire ou ramifié, contenant de 1 à 6 atomes de carbone environ,
- R₁₃ désigne un atome d'hydrogène ou un radical -SO₃H,
- l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de composé de formule (III) : le composé dans lequel X désigne un radical -NH-, R₁₁ désigne un radical -SO₃H, R₁₂ et R₁₃ désignent tous deux un atome d'hydrogène, c'est-à-dire l'acide 2-[4-(camphométhylidène) phényl] benzimidazole-5-sulfonique.

Les composés de structures (I), (II), (III) sont décrits dans le brevet US 4.585.597 et les brevets FR 2.236.515, 2.282.426, 2.645.148, 2.430.938, 2.592.380.

On peut citer comme autres exemples de dérivés du benzylidène camphre utilisables dans l'invention les composés de formule générale (b) suivante : 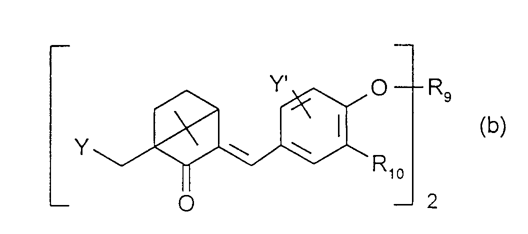 dans laquelle :
- R₉ désigne un radical divalent : -(CH₂)ₘ- ou -CH₂ -CHOH-CH₂-, m étant un nombre entier allant de 1 à 10 (1 ≤ m ≤ 10),
- R₁₀ désigne un atome d'hydrogène, un radical alcoxy contenant de 1 à 4 atomes de carbone environ ou un radical divalent - O - relié au radical R₉ lorsque celui-ci est divalent lui aussi,
- Y et Y' désignent un atome d'hydrogène ou un radical -SO₃H, au moins un de ces radicaux Y ou Y' est différent de l'hydrogène. Là encore la fonction -SO₃H peut être neutralisée.

On peut citer comme exemples particuliers les composés suivants de formule (b) dans laquelle Y représente -SO₃H, Y' est -H, R₁₀ est H et R₉ est -CH₂-CH₂-, c'est à dire l'acide éthylène bis [(4'-oxy benzylidène) 3-campho-10 sulfonique].

Selon l'invention, la quantité de dérivés d'ose de l'acide ascorbique est par exemple choisie de 0,05 % à 20 % en poids par rapport au poids total de la composition et de préférence de 0,5 % à 10 %. et mieux de 0,5 % à 5 %. De plus, la composition peut contenir un ou plusieurs dérivés d'ose.

De même, la quantité de filtre UVA utilisable dans l'invention est celle généralement utilisée dans les domaines concernés. En pratique, on utilise de 0,1 % à 10 % en poids par rapport au poids total de la composition et de préférence de 0,1 % à 5 %. De plus, la composition peut contenir un ou plusieurs filtres UVA hydrosolubles.

Les huiles utilisables dans l'invention sont celles généralement utilisées dans les domaines concernés. Elles peuvent être végétales, minérales ou synthétiques, éventuellement siliconées et/ou fluorées.

L'invention peut également contenir des adjuvants hydrophiles ou lipophiles comme des gélifiants, des conservateurs, des opacifiants, des émulsionnants, des coémulsionnants, des parfums et leurs solubilisants ou peptisants, des colorants, des pigments, des charges, ainsi que des actifs lipophiles ou hydropliles autres que l'ester d'ose de l'acide ascorbique et le filtre UVA.

Les quantités d'huile et d'eau sont généralement celles utilisées dans les domaines considérés et sont fonction de la forme galénique de la composition. Pour une émulsion huile-dans-eau ou une dispersion d'huile dans de l'eau par des sphérules lipidiques, l'huile peut représenter de 2 % à 40 % en poids par rapport au poids total de la composition.

De même les adjuvants sont utilisés en quantité habituelle et peuvent représenter, au total, de 0,1 % à 20 % en poids. Leur quantité est fonction de leur nature.

La composition de l'invention peut être appliquée par voie topique sur toutes les parties du corps et du visage.

L'invention a aussi pour objet une utilisation de la composition définie ci-dessus pour le traitement cosmétique des rides et/ou des ridules de la peau ainsi qu'une utilisation de cette composition pour tonifier, hydrater et/ou raffermir la peau.

L'invention a encore pour objet une utilisation de la composition définie ci-dessus pour le traitement cosmétique des taches cutanées dues au vieillissement, présentes sur le visage et/ou le corps, y compris les mains et le cuir chevelu ainsi que pour la préparation d'une crème destinée au traitement des taches de la peau d'origine pathologique.

L'invention a encore pour objet un procédé de traitement cosmétique de la peau, consistant à appliquer la composition définie ci-dessus sur la peau.

Les tests suivants ont mis en évidence les avantages de la présente invention. Le but des tests a été de montrer l'aptitude des compositions de l'invention à atténuer efficacement les rides et les ridules, modifier le teint de la peau qui apparaît plus rosé, effacer les taches de pigmentation, supprimer les squames et donner une consistance plus élastique à la peau. Elle permet une protection efficace de la peau contre les rayons solaires ainsi qu'un blanchiment de la peau.

Les tests ont été effectués sur un panel expert composé de 15 individus. La crème a été appliquée pendant plusieurs jours à raison d'une application par jour. Le panel a répondu sur les critères suivants : meilleure mine, teint moins brouillé et peau plus lisse. Les résultats sont les suivants :
- 9 personnes sur 15 ressentent un effet très important, important ou moyennement important concernant la sensation d'avoir une meilleure mine, la sensation d'avoir le teint moins brouillé ainsi que la sensation d'avoir une peau plus lisse.

D'autres caractéristiques et avantages de l'invention ressortiront mieux de la description qui suit, donnée à titre illustratif. Dans les exemples ci-après de compositions cosmétiques et/ou dermatologiques conformes à l'invention, les compositions sont données en % pondéral.

### EXEMPLE 1 : Crème huile-dans-eau pour la prévention de le pigmentatation de la peau

| **Composition** | | |
|---|---|---|
| *A*_{*1*} | - Tri-stéarate de Sorbitane (émulsionnant) | 0,7 % |
| | - Stéarate de polyéthylène glycol (40.OE) (émulsionnant) | 1,6 % |
| | - Alcool cétylique (co-émulsionnant) | 3,2 % |
| | - Mono,di,tri-palmitostéarate de glycéryle (émulsionnant) | 2,4 % |
| | - Myristate de myristyle (huile) | 2 % |
| | - Fraction liquide de beurre de karité (huile) | 2 % |
| | - Conservateur) | 0,2 |
| *A*_{*2*} | - Cyclopentadiméthylsiloxane (huile) | 15 % |
| *B* | - Eau déminéralisée | qsp 100 % |
| | - Glycérol (hydratant) | 3 % |
| | - Ascorbyl-2glucoside vendu par Hayashibara | 1 % |
| | - Conservateur | 0,2% |
| *C* | - Acide téréphtalylidène di-camphosulfonique dans l'eau à 33 % | 2 % |
| | - Triéthanolamine (neutralisant) | 0,3 % |
| *D* | - Parfum | 0,3 % |

### Préparation de la phase A₁ + A₂

Les constituants de A₁ sont solubilisés à 80 °C. Lorsque le mélange est limpide, la température est abaissée à 65 °C et on ajoute A₂. Le mélange doit être limpide et homogène. On maintient la température de 65 °C.

### Fabrication

Dans un bécher de fabrication, les constituants de B sont solubilisés à 85 °C-90 °C. Après vérification de la limpidité, la température est ramenée à 65 °C. On réalise l'émulsion, sous agitation, en versant (A₁+A₂) dans B. On poursuit le refroidissement sous agitation. A 40 °C on ajoute la phase C, puis on maintient l'agitation. Enfin, on ajoute le parfum et on laisse refroidir à 20 °C sous agitation.

### EXEMPLE 2 : Crème huile-dans-eau anti-taches

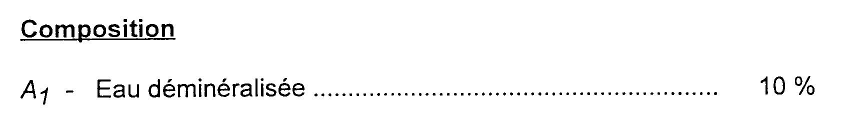

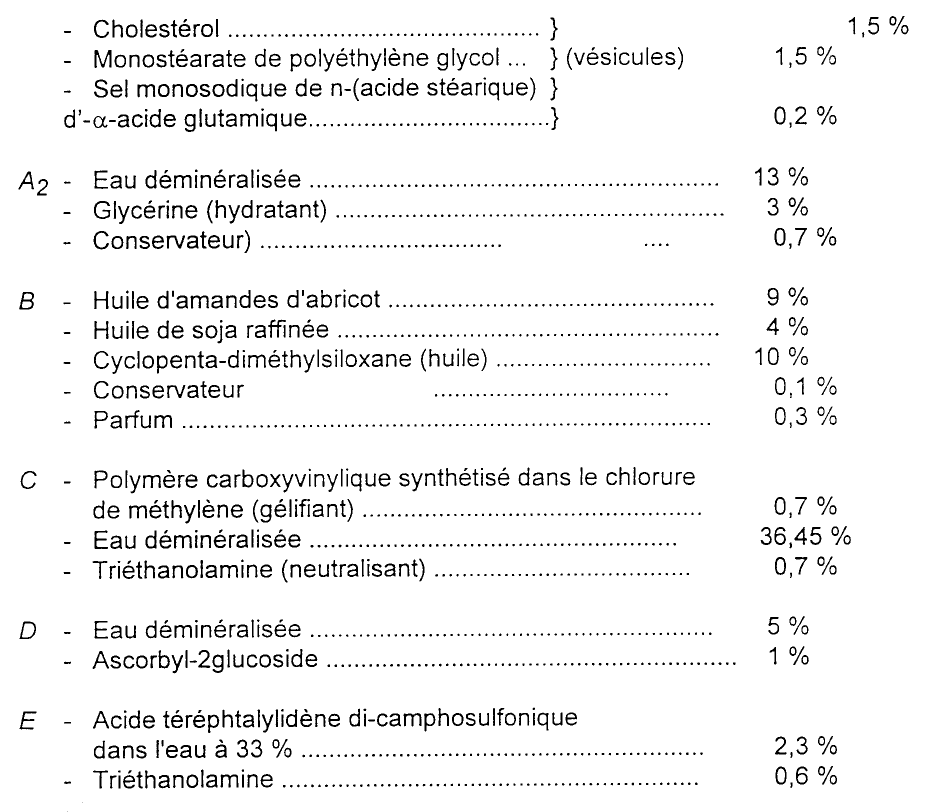

### Fabrication

On fait fondre les constituants de A₁ à 100 °C. On laisse gonfler sous agitation pendant 1H30. Lorsque le mélange est homogène on ajoute A₂ ; on stabilise la température à 80 °C. On effectue alors deux passages à l'homogénéisateur haute pression pour former les vésicules.

On prépare B à 70 °C le mélange doit être limpide. On refroidit à 50 °C. On ajoute B dans A à 50 °C. On fait alors deux passages à l'homogénéisateur haute pression pour disperser la phase grasse B. On refroidit à 30 °C. On ajoute C (le gel aura été préalablement préparé dans l'eau à 80 °C, en saupoudrant le polymère carboxyvinylique ; après gonflement de ce dernier, on neutralise à la triéthanolamine sous agitation ; le gel doit être bien lisse). On ajoute D puis E. On maintient l'agitation pendant 5 minutes. La fabrication est terminée.

### EXEMPLE 3 : Gel protecteur contre les rayons solaires

| **Composition** | | |
|---|---|---|
| *A* | - Eau déminéralisée | qsp 100 % |
| | - Glycérine | 3 % |
| | - Para-hydroxybenzoate de méthyle | 0,2 % |
| | - Ascorbyl-2glucoside | 1 % |
| | - Gomme de Xanthane (épaississant) | 0,2 % |
| *B* | - Parsol® MCX (filtre UVB) | 4 % |
| | - Alkyl benzoate (Finsolv TN, Société Witco) | 4 % |
| | - Polymère carboxyvinylique alkylé (Pemulen TR 2, Société Goodrich) | 0,45 % |
| | - Triéthanolamine | 0,45 % |
| *C* | - Acide téréphtalylidène di-camphosulfonique dans l'eau à 33 % | 2,3 % |
| | - Triéthanolamine | 0,6 % |

### Fabrication

On prépare la phase A en saupoudrant, sous agitation le gélifiant dans l'eau contenant les ingrédients dissouts. On émulsionne en incorporant la phase B à la phase A, sous forte agitation. On lisse et on laisse refroidir sous agitation à pales lentes. A 35 °C, on ajoute C. On laisse refroidir à 25 °C. La fabrication du gel est terminée.

### EXEMPLE 4 : Lotion « teint clair »

| **Composition** | | |
|---|---|---|
| *A* | - Triglycérides ricinoléiques hydrogénés oxyéthylénés (60 OE) (peptisant) | 0,09 % |
| | - Parfum | 0,03 % |
| *B* | - Eau déminéralisée | qsp 100 % |
| | - Glycérine | 5,5 % |
| | - Ascorbyl-2 glucoside | 1 % |
| | - Conservateur | 0,3 % |
| *C* | - Acide téréphtalylidène di-camphosulfonique dans l'eau à 33 % | 2,3 % |
| | - Triéthanolamine | 0,6 % |

### Fabrication

On mélange les constituants de A à 40 °C. Lorsqu'ils sont parfaitement solubilisés, on ajoute successivement les constituants de B à température ambiante. On maintient l'agitation et on vérifie la bonne solubilisation des constituants. On ajoute C ; le mélange doit être limpide. La fabrication est terminée.

## Revendications

1. Composition pour prévenir et/ou lutter contre les taches et/ou le vieillissement cutanés, caractérisée en ce qu'elle contient au moins un filtre UVA sulfonique hydrosoluble et au moins un ester d'ose de l'acide ascorbique compatible avec ledit filtre, dans un milieu cosmétiquement et/ou dermatologiquement acceptable.

2. Composition selon la revendication 1, caractérisée en ce que le filtre est choisi dans le groupe constitué des dérivés sulfonés ou sulfonatés du benzylidène camphre.

3. Composition, selon la revendication 1 ou 2, caractérisée en ce que le filtre présente la formule (I) suivante : 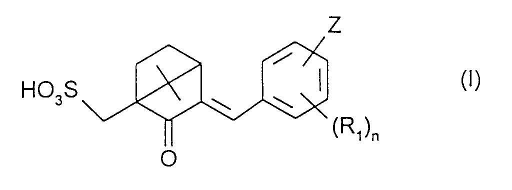 dans laquelle :
- Z désigne un groupement 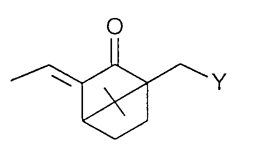 dans lequel Y représente -H ou -SO₃H, éventuellement neutralisé,
- n est égal à 0 ou est un nombre allant de 1 à 4 (0 ≦n≦ 4),
- R₁, représente un ou plusieurs radicaux alkyle ou alkoxy, identiques ou différents, linéaires ou ramifiés, contenant de 1 à 4 atomes de carbone.

4. Composition selon l'une quelconque des revendications 1 à 3, caractérisée en ce que le filtre est l'acide benzène 1,4 [di(3-méthylidène campho-10-sulfonique)].

5. Composition selon l'une quelconque des revendications précédentes, caractérisée en ce que l'ester d'ose de l'acide ascorbique est l'ascorbyl-2-glucoside.

6. Composition selon l'une quelconque des revendications précédentes, caractérisée en ce que qu'elle se présente sous forme d' une émulsion huile-dans-eau ou sous forme d'une dispersion de sphérules lipidiques.

7. Composition selon l'une quelconque des revendications précédentes, caractérisée en ce que l'ester d'ose de l'acide ascorbique représente de 0,05 % à 20 % en poids par rapport au poids total de la composition.

8. Composition selon l'une quelconque des revendications précédentes, caractérisée en ce que le filtre UVA représente de 0,1 % à 10 % en poids par rapport au poids total de la composition.

9. Composition selon l'une quelconque des revendications précédentes, caractérisée en ce que'elle contient aussi des adjuvants hydrophiles ou lipophiles.

10. Composition selon l'une quelconque des revendications précédentes, caractérisée en ce que les adjuvants sont choisis parmi les gélifiants, les conservateurs, les parfums, les charges, les matières colorantes, les actifs autres que l'ester d'ose de l'acide ascorbique et le filtre UVA.

11. Utilisation cosmétique de la composition selon l'une quelconque des revendications précédentes pour traiter les taches cutanées dues au vieillissement.

12. Utilisation cosmétique de la composition selon l'une quelconque des revendications 1 à 10, pour traiter les rides et/ou les ridules de la peau.

13. Utilisation de la composition selon l'une quelconque des revendications 1 à 10 pour tonifier, hydrater et/ou raffermir la peau.

14. Utilisation de la composition selon l'une quelconque des revendications 1 à 10 pour préparer une crème destinée au traitement des maladies de peau conférant à cette dernière des taches.

15. Procédé de traitement cosmétique de la peau, caractérisé en ce qu'il consiste à appliquer sur la peau une composition selon l'une quelconque des revendications 1 à 10.

## Claims

1. Composition for preventing and/or combating skin marks and/or ageing of the skin, characterized in that it contains at least one water-soluble sulphonic UVA screening agent and at least one saccharide ester of ascorbic acid which is compatible with the said screening agent, in a cosmetically and/or dermatologically acceptable medium.

2. Composition according to Claim 1, characterized in that the screening agent is chosen from the group consisting of sulphone-containing or sulphonate-containing benzylidenecamphor derivatives.

3. Composition according to Claim 1 or 2, characterized in that the screening agent has the following formula (I): 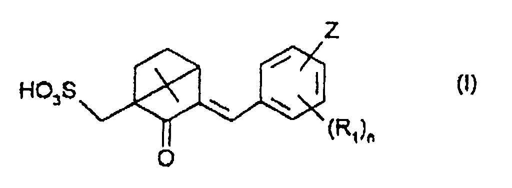 in which:
- Z denotes a group 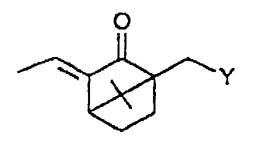 in which Y represents -H or -SO₃H, optionally neutralized,
- n is equal to 0 or is a number ranging from 1 to 4 (0≤n≤4),
- R₁ represents one or more linear or branched alkyl or alkoxy radicals, which may be identical or different, containing from 1 to 4 carbon atoms.

4. Composition according to any one of Claims 1 to 3, characterized in that the screening agent is benzene-1,4-[di(3-methylidene-10-camphorsulphonic)] acid.

5. Composition according to any one of the preceding claims, characterized in that the saccharide ester of ascorbic acid is ascorbyl-2-glucoside.

6. Composition according to any one of the preceding claims, characterized in that it is in the form of an oil-in-water emulsion or in the form of a dispersion of lipid spherules.

7. Composition according to any one of the preceding claims, characterized in that the saccharide ester of ascorbic acid represents from 0.05 % to 20 % by weight relative to the total weight of the composition.

8. Composition according to any one of the preceding claims, characterized in that the UVA screening agent represents from 0.1 % to 10 % by weight relative to the total weight of the composition.

9. Composition according to any one of the preceding claims, characterized in that it also contains hydrophilic or lipophilic adjuvants.

10. Composition according to any one of the preceding claims, characterized in that the adjuvants are chosen from gelling agents, preserving agents, fragrances, fillers, dyestuffs, and active agents other than the saccharide ester of ascorbic acid and the UVA screening agent.

11. Cosmetic use of the composition according to any one of the preceding claims for treating skin marks due to ageing.

12. Cosmetic use of the composition according to any one of Claims 1 to 10 for treating wrinkles and/or fine lines on the skin.

13. Use of the composition according to any one of Claims 1 to 10 for toning, moisturizing and/or firming up the skin.

14. Use of the composition according to any one of Claims 1 to 10 for preparing a cream intended for treatment of skin diseases which leave marks on the skin.

15. Process for the cosmetic treatment of the skin, characterized in that it consists in applying to the skin a composition according to any one of Claims 1 to 10.

## Patentansprüche

1. Zusammensetzung zur Vermeidung oder Bekämpfung von Flecken oder der Hautalterung, dadurch gekennzeichnet, daß sie mindestens ein wasserlösliches UV-A-Filter mit Sulfonsäuregruppen und mindestens einen Zuckerester von Ascorbinsäure, der mit dem Filter kompatibel ist, in einem kosmetisch oder dermatologisch akzeptablen Medium enthält.

2. Zusammensetzung nach Anspruch 1, dadurch gekennzeichnet, daß das Filter unter Sulfonsäurederivaten und Sulfonatderivaten von Benzylidencampher ausgewählt ist.

3. Zusammensetzung nach Anspruch 1 oder 2, dadurch gekennzeichnet, daß das Filter die folgenden Formel (I) 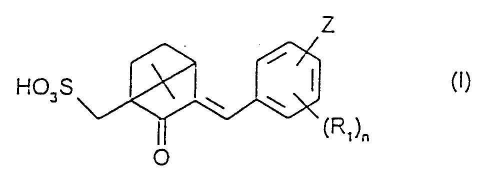 aufweist, in der bedeuten:
- Z eine Gruppe 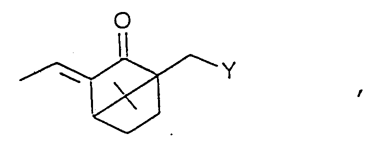 in der Y -H oder eine Gruppe -SO₃H, die gegebenenfalls neutralisiert ist, darstellt,
- n 0 oder eine Zahl von 1 bis 4 (0 ≤ n ≤ 4)
- R₁ eine oder mehrere gleiche oder verschiedene, geradkettige oder verzweigte Alkyl- oder Alkoxygruppen, die 1 bis 4 Kohlenstoffatome enthalten.

4. Zusammensetzung nach einem der Ansprüche 1 bis 3, dadurch gekennzeichnet, daß es sich bei dem Filter um Benzol-1,4-di-[3-methylidencampher-10-sulfonsäure] handelt.

5. Zusammensetzung nach einem der vorhergehenden Ansprüche, dadurch gekennzeichnet, daß der Zuckerester der Ascorbinsäure das Ascorbyl-2-glucosid ist.

6. Zusammensetzung nach einem der vorhergehenden Ansprüche, dadurch gekennzeichnet, daß sie in Form einer Öl-in-Wasser-Emulsion oder einer Dispersion von Lipidkügelchen vorliegt.

7. Zusammensetzung nach einem der vorhergehenden Ansprüche, dadurch gekennzeichnet, daß der Zuckerester der Ascorbinsäure 0,05 bis 20 Gew.-%, bezogen auf das Gesamtgewicht der Zusammensetzung, ausmacht.

8. Zusammensetzung nach einem der vorhergehenden Ansprüche, dadurch gekennzeichnet, daß das UV-A-Filter 0,1 bis 10 Gew.-%, bezogen auf das Gesamtgewicht der Zusammensetzung, ausmacht.

9. Zusammensetzung nach einem der vorhergehenden Ansprüche, dadurch gekennzeichnet, daß sie außerdem hydrophile oder lipophile Hilfsstoffe enthält.

10. Zusammensetzung nach einem der vorhergehenden Ansprüche, dadurch gekennzeichnet, daß die Hilfsstoffe unter Gelbildnern, Konservierungsmitteln, Parfüms, Füllstoffen, Färbemitteln und unter Wirkstoffen, die von dem Zuckerester der Ascorbinsäure und dem UV-A-Filter verschieden sind, ausgewählt sind.

11. Kosmetische Verwendung der Zusammensetzung nach einem der vorhergehenden Ansprüche für die Behandlung von Hautflecken, die durch die Alterung hervorgerufen werden.

12. Kosmetische Verwendung der Zusammensetzung nach einem der Ansprüche 1 bis 10 für die Behandlung von Falten und/oder Fältchen der Haut.

13. Verwendung der Zusammensetzung nach einem der Ansprüche 1 bis 10 für die Belebung, die Hydratisierung oder die Straffung der Haut.

14. Verwendung der Zusammensetzung nach einem der Ansprüche 1 bis 10 für die Herstellung einer Creme, die für die Behandlung von Hautkrankheiten, durch die Flecken auf der Haut verursacht werden, bestimmt ist.

15. Verfahren zur kosmetischen Behandlung der Haut, dadurch gekennzeichnet, daß auf die Haut eine Zusammensetzung nach einem der Ansprüche 1 bis 10 aufgetragen wird.
